## Europäisches Patentamt

## European Patent Office

## Office européen des brevets

(19)

(11) Numéro de publication: **0 005 703**
**B1**

(12)

# FASCICULE DE BREVET EUROPEEN

(45) Date de publication du fascicule de brevet: **14.10.81**

(21) Numéro de dépôt: **78400012.7**

(22) Date de dépôt: **05.06.78**

(51) Int. Cl.³: **C 12 P 7/10**, C 10 L 1/00,
C 05 F 9/00, C 05 F 7/00,
A 23 K 1/00, C 02 F 1/00

(54) **Procédé de préparation de combustible et de substances nutritives à partir de déchets.**

(43) Date de publication de la demande:
**12.12.79 Bulletin 79/25**

(45) Mention de la délivrance du brevet:
**14.10.81 Bulletin 81/41**

(84) Etats Contractants Désignés:
**BE CH DE FR GB LU NL SE**

(56) Documents cités:

DE - A - 811 821
FR - A - 638 690
FR - A - 984 983
FR - A - 2 119 987
FR - A - 2 321 469
US - A - 2 198 785
US - A - 2 312 196
US - A - 3 711 392
US - A - 3 725 538
US - A - 3 787 583
US - A - 3 848 813
NL - A - 74 06786

CHEMICAL ABSTRACTS, vol. 86, 1977,
104.478d, page 348 Columbus, Ohio, USA
T. OYAMOTO et al.: "Single cell protein or
alcohol production from organic waste,
especially from kitchen garbage".

(73) Titulaire: **John Lester Lang**
**P.O. Box 1242**
**Midland, Michigan 48640 (US)**

(72) Inventeur: **John Lester Lang**
**P.O. Box 1242**
**Midland, Michigan 48640 (US)**

(74) Mandataire: **Peuscet, Jacques**
**3, Square de Maubeuge**
**F-75009 Paris (FR)**

Courier Press, Leamington Spa, England.

# Procédé de préparation de combustible et de substances nutritives à partir de déchets

On sait que le problème qui se pose dans le traitement destiné à l'élimination des déchets ou ordures municipales concerne la phase finale de l'élimination des matériaux nocifs qui y sont contenus.

L'élimination finale des déchets liquides, notamment des déchets municipaux (ou eaux d'égouts) est un problème coûteux et embarrassant.

Simultanément, il existe une pénurie en pétrole due aux prix élevés de celui-ci tandis qu'il existe une quantité d'énergie importante qui est stockée dans des déchets tels que les ordures ménagères, les boues des eaux d'égouts et d'autres matériaux qui sont désignés sous le terme général de "bio-masse"; ces matériaux contiennent d'importantes proportions de cellulose et d'hydrates de carbone similaires qui peuvent être converties, par hydrolyse, en des sucres fermentescibles, notamment comme décrit dans le présent procédé, et ces sucres peuvent être ensuite convertis en alcool.

Le problème posé par l'élimination de déchets municipaux solides (ou ordures ménagères) est présentement résolu par la technique dite "d'enfouissement sanitaire", d'incinération, d'élimination dans des mines abandonnées, initialement dans des lieux de décharge etc.

Ces méthodes entraînent des gaspillages en ce qui concerne la perte d'énergie de ces matériaux, la dissémination de ressources intéressantes et, dans le cas de l'incinération, la perte d'énergie emmagasinée (sous forme de composés qui fixent l'azote) réprésente un gaspillage formidable car ces matériaux doivent être à nouveau synthétisés soit par la nature soit par l'homme afin de fournir les aliments nécessaires à la race humaine.

On a déjà prévu dans le brevet US 2 198 785 un procédé de traitement de déchets pouvant avoir subi préalablement une séparation visant à extraire les métaux, les eaux et le verre, ces déchets étant soumis à une hydrolyse par un acide, après quio le produit hydrolysé ne peut être traité par fermentation pour la production d'alcool.

On a également déjà prévu dans le brevet français 2 321 469 de stériliser des matières solides en milieu éventuellement acide et d'inoculer le mélange stérile par une espèce fermentaire pour provoquer une fermentation suivie d'une distillation.

Le procédé selon le brevet américain précité donne des résultats variables et l'invention se propose donc de perfectionner les procédés de préparation de matériaux utiles comprenant un combustible en améliorant, à un prix de revient peu élevé, les performances industrielles de ces procédés, sans nécessiter d'étape coûteuse particulière telle que la stérilisation.

La présente invention concerne un procédé d'élimination des déchets tels que des eaux d'égouts, des ordures ménagères etc., procédé dans lequel les résidus sont, après en avoir éliminé les matériaux recyclables, tels que du verre, des métaux, des matières plastiques, des os, etc., hydrolysés par de l'anhydride sulfureux (agent hydrolytique dans l'eau) pour convertir la majeure partie des composés hydrolysables en des substances fermentescibles puis en un carburant tel que l'alcool éthylique.

La partie non fermentescible est récupérée de façon convenable, conjointement avec les micro-organismes en excès qui provoquent sa fermentation et sa transformation en un matériau utilisable, par exemple comme éléments nutritifs d'origine animale ou végétale et comme autres produits utiles.

La présente invention a donc pour but de fournir un moyen permettant à la fois l'élimination finale des déchets d'un carburant liquide, de substances nutritives riches en matières azotées, et de l'eau purifiée.

Un autre but de la présente invention est de réaliser un combustible liquide à partir de déchets cellulosiques (par exemple, à partir de la "biomasse").

Un autre but de la présente invention est de réaliser un combustible liquide destiné à être utilisé dans un moteur à combustion interne.

Comme agent qui favorise l'étape d'hydrolyse et de saccharification, on utilise l'acide sulfureux.

L'hydrolyse et la saccharification sont accélérées en réglant de façon appropriée la température, le type et la quantité de l'agent hydrolytique et la pression concomitante nécessaire pour maintenir la température choisie dans l'étape d'hydrolyse et de saccharification.

Les exemples non limitatifs suivent illustrent la présente invention.

### Exemple 1

On prépare de façon "synthétique" des déchets municipaux solides en mélangeant des graisses, des déchets de viande, du papier, des feuilles de choux, des bouts de carottes, des pelures de pommes de terre, des os, des pellicules de polyéthylène, des feuilles d'aluminium, du verre, du métal de boites de conserve, des bouchons de bouteilles et du cuir.

On peut récupérer les matériaux magnétiques contenus dans ce déchet solide synthétique lorsqu'on le fait passer dans un séparateur magnétique. Les matériaux non magnétiques qui sont ainsi séparés donnent, après passage dans un broyeur à rouleaux, un matériau friable dont la taille a subi une réduction considérable et qui comprend la plupart des matériaux d'origine végétale ou animale, les matières plastiques cassantes, des os et du verre; ce broyeur à rouleaux aplatit simplement les matériaux non magnétiques, les pellicules flexibles en matière plastique et les constituants les plus durs tels que le cuir, le cartilage, etc . . . de ces déchets.

Lorsqu'on le fait passer à travers un tamis grossier, on sépare du matériau broyé, une partie qui contient les métaux non magnétiques, les matières plastiques flexibles, et les constituants durs tels que le cuir, le papier, etc . . . , et une autre partie qui contient des matériaux moins durs d'origine animale ou végétale, des matières plastiques friables, des os, du verre, etc . . .

La partie non friable, lorsqu'elle est soumise à une flottation dans de l'eau salée de 1,2 de densité est séparée en deux fractions, la fraction qui est récupérée au fond contenant les métaux non magnétiques qui sont ainsi récupérés. La fraction surnageante est soumise à un trempage pendant au moins quatre heures; les matériaux ayant de l'affinité pour l'eau tels que le cuir, le papier, des tissus, absorbent l'eau et coulent. Une nouvelle opération de flottation permet de séparer les matériaux hydrophobes tels que des pellicules en matière plastique, du caoutchouc, etc . . .

La fraction surnageante est utilisée comme combustible solide et récupérée comme désirée tandis que l'autre fraction est séparée et contient du cuir, du cartilage, du papier etc. que l'on fait passer à travers un broyeur fin du type déchiqueteur.

Le matériau friable provenant du broyeur à rouleaux est, après tamisage, soumis à une séparation par flottation en utilisant de l'eau; on obtient ainsi une boue contenant la majeure partie des composés d'origine végétale et animale des déchets d'origine et un mélange, que l'on obtient par dépôt, de matières friables telles que des os, du verre, des matières plastiques etc . . .

La boue de matières d'origine animale et végétale est envoyée dans un broyeur fin du type déchiqueteur tandis que le matériau déposé est séparé par flottation par gravité pour donner des granules de matière plastique friables, des granules d'os et des fragments de verre.

Après le fin broyage de ce matériau séparé d'origine animale et végétale, le mélange est soumis à une opération d'hydrolyse mettant en oeuvre de l'acide sulfureux comme catalyseur.

Le matériau saccharifié est refroidi et concentré en le faisant passer à travers un évaporateur que l'on fait fonctionner de façon que la température de l'effluent soit de 65°C et que la teneur en matières solides organiques soit d'environ 30%. On règle le pH à une valeur de 3,4 et on ajoute une faible quantité d'enzymes, par exemple sous forme d'orge maltée. Ce mélange, qui contient des enzymes, ets maintenu à 65°C pendant 3 heures.

Après refroidissement à 27°C et un nouveau réglage du pH à une valeur de 3,4, on ajoute de la levure et on maintient la température et le pH jusqu'a ce que la fermentation cesse. Le matériau fermenté est filtré, le résidu est stérilisé à la vapeur et stocké. On distille le filtrat en utilisant une colonne de 1,20 m de hauteur garnie de fragments de porcelaine et équipée d'une tête de prélèvement qui est réglée à un taux — de reflux de 20/l. Le chauffage du ballon de la colonne est réglé de façon à maintenir la température au voisinage de 76—80°C dans le ballon.

Par rapport à une charge initiale de 100 parties dans le ballon, le distillat contient environ 11 parties d'un mélange éthanol -eau. L'éthanole est, éventuellement, séché par de l'oxyde de calcium, filtré et mélangé dans des proportions 1/9 avec de l'essence. Ce mélange de liquide combustible, après des réglages mineurs du carburateur, peut brûler proprement et sans difficulté dans un moteur à combustion interne de type classique.

Le résidu filtré provenant de l'étape de fermentation est à nouveau combiné avec le résidu du ballon de la colonne de distillation, le tout étant évaporé jusqu'au moment où un sirop épais se forme, de l'eau distillée étant produite comme sous-produit. On combine ce sirop avec des grains de nourriture broyés, le sirop faisant à la fois office de liant pour la mise en boulettes, et apportant simultanément les éléments nutritifs cuits, stérilisés des déchets municipaux et les éléments nutritifs des levures contenues dans le résidu de fermentation, ces levures étant, bien entendu, multipliées pendant l'étape de fermentation.

### Exemple 2

Trois litres des boue activée partiellement déshydratée obtenue à partir de déchets soumis à des opérations de séparation et de broyage, et ayant une teneur totale en matière solide de 8%, sont placés dans un récipient pressurisé résistant à la corrosion muni d'un agitateur, d'un élément de chauffage électrique et d'un logement de thermocouple. Le pH de la boue est abaissé à une valeur de 1,5 par addition d'ions d'acide sulfureux. Après avoir fermé de façon étanche le réacteur et commencé l'agitation, on règle le système de chauffage électrique de façon à maintenir une température de 140°C; on poursuit l'agitation et le chauffage pendant 3,5 heures.

On décharge ensuite le contenu du réacteur dans chambre d'expansion et on y injecte de la vapeur, le pH étant réglé à 3,4. Après refroidissement à 65°C, on ajoute des enzymes sous forme d'orge maltée et on maintient les conditions de température et de pH pendant quatre heures. On laisse la température à 27°C et on ajuste le pH à 3,4. On ajoute de la levure et on commence la fermentation. Les conditions de fermentation sont maintenues jusqu'à ce que la fermentation cesse.

La matériau fermenté est filtré, et le résidu est stérilisé à la vapeur et stocké pour un usage ultérieur. Le distillat donne, pour 100 parties de filtrat, 3,4 parties d'un azéotrope ethanol-eau contenant 95% d'éthanol. Un mélange de ce produit dans les proportions de 1/9 avec de l'essence ordinaire brûle bien et proprement dans un moteur à combustion interne conventionnel, après un réglage mineur du carburateur.

**Revendications**

1. Procédé de préparation de matériaux utiles comprenant un combustible liquide à partir de déchets solides comprenant une bio-masse, ce procédé consistant à soumettre les déchets à une séparation, à soumettre la bio-masse à un broyage puis à une action d'hydrolyse acide à une température et à une pression suffisante pour que la partie organique des déchets soit sensiblement solubilisée et convertie en sucres fermentescibles, et à faire fermenter le produit résultant pour provoquer la formation d'alcool et l'obtention de résidus à usage nutritif, caractérisé par le fait que:

a) on réalise l'hydrolyse en présence d'acide sulfureux comme catalyseur d'hydrolyse;

b) on filtre le produit résultant fermenté pour séparer un filtrat et un résidu;

c) on distille le filtrat, comme connu en soi, pour obtenir un combustible liquide et un résidu.

2. Procédé selon la revendication 1, caractérisé par le fait que la séparation, à laquelle sont soumis les déchets solides, comporte:

a) une séparation des matériaux métalliques;

b) une séparation des matériaux friables et non friables, les matériaux friables étant principalement constitués de matières plastiques cassantes, d'os et de verre et contenant la bio-masse et les matériaux non friables étant principalement constitués de métaux non magnétiques, de cuir, de papier, de tissu, de cartilage, de pellicules de matières plastiques et de caoutchouc;

c) une séparation des matériaux friables précitée et de la bio-masse, par flottation dans l'eau, cette séparation provoquant un trempage dans l'eau du matériau hydrolysable constituant la bio-masse.

3. Procédé selon la revendication 2, caractérisé par le fait que la séparation des matériaux métalliques mentionnée à l'étape a) de la revendication 2 comprend une séparation magnétique permettant de récupérer les métaux magnétiques.

4. Procédé selon l'une des revendications 2 ou 3, caractérisé par le fait que l'étape b) de la revendication 2 est réalisée par broyage puis tamisage des déchets, les matériaux friables traversant le tamis alors que les matériaux non friables ne le traversent pas.

5. Procédé selon l'une des revendications 1 à 4, caractérisé par le fait que le résidu de l'étape b) de la revendication 1 est soumis à une stérilisation avant d'être utilisé pour fabriquer un produit nutritif.

6. Utilisation du résidu de l'étape b) de la revendication 1 et/ou du résidu de l'étape c) de la revendication 1 pour fabriquer un aliment pour animaux ou pour végétaux.

7. Utilisation du combustible liquide de l'étape c) de la revendication 1 comme combustible de moteur à combustion interne.

**Patentansprüche**

1. Verfahren zur Herstellung von nützlichen Stoffen, die einen flüssigen Brennstoff enthalten, aus festen Abfällen enthaltend eine Bio-masse, wobei man die Abfälle einer Filtration unterwirft, die Biomasse einer Zerkleinerung und nachher einer sauren Hydrolyse bei einer ausreichenden Temperatur und Druck unterwirft, um den organischen Teil der Abfälle erheblich zu solubilisieren und in vergärbare Zucker umzuwandeln, und das erhaltene Produkt einer Gärung unterwirft, um die Bildung von Alkohol und eines für Nährzwecke geeigneten Rückstandes herbeizuführen, dadurch gekennzeichnet, daß man

a) die Hydrolyse in Gegenwart von Schwefelsäure als Hydrolysekatalysator durchführt,

b) das durch Gärung erhaltene Produkt filtriert und in ein Filtrat und einen Rückstand trennt und

c) das Filtrat in an sich bekannter Weise destilliert, um einen flüssigen Brennstoff und einen Rückstand zu erhalten.

2. Verfahren nach Anspruch 1, dadurch gekennzeichnet, daß die Trennung, der die festen Abfälle unterworfen werden, einschließt:

a) eine Abtrennung der metallischen Materialien,

b) eine Trennung der zerreibbaren und der nicht-zerreibbaren Materialien, wobei die zerreibbaren Materialien hauptsächlich aus spröden Kunststoffen, Knochen und Glas bestehen und die Biomasse enthalten und die nicht-zerreibbaren Materialien hauptsächlich aus unmagnetischen Metallen, Leder, Papier, Geweben, Knorpel und Filmen aus Kunststoffen und Kautschuk bestehen,

c) eine Trennung der vorstehend genannten zerreibbaren Materialien und der Biomasse durch Flottation im Wasser, wobei bei dieser Trennung ein Eintauchen des hydrolisierbaren Materials, das die Biomasse darstellt, in Wasser herbeigeführt wird.

3. Verfahren nach Anspruch 2, dadurch gekennzeichnet, daß die Abtrennung der metallischen Materialien gemäß Stufe a) von Anspruch 2 eine magnetische Trennung einschließt, die eine Wiedergewinnung der magnetischen Materialien gestattet.

4. Verfahren nach einem der Ansprüche 2 oder 3, dadurch gekennzeichnet, daß die Stufe b) eine Zerkleinerung und danach ein Absieben der Abfälle vorsieht, wobei die zerreibbaren Materialien durch das Sieb hindurchgehen und die nicht-zerreibbaren Materialien nicht hindurchgehen.

5. Verfahren nach einem der Ansprüche 1—4, dadurch gekennzeichnet, daß der Rückstand der Stufe b) von Anspruch 1 einer Sterilisation unterworfen wird, bevor er zur Herstellung eines für Nährzwecke geeigneten Produktes verwendet wird.

6. Verwendung des Rückstandes der Stufe b) von Anspruch 1 und/oder des Rückstandes von Stufe c) von Anspruch 1 zur Herstellung eines Futtermittels für Tiere oder für Pflanzen.

7. Verwendung des flüssigen Brennstoffs von Stufe c) von Anspruch 1 als Treibstoff für einen Motor mit innerer Verbrennung.

## Claims

1. Process for the preparation of useful materials, comprising a liquid fuel, from solid waste comprising a biomass, this process consisting in subjecting the waste to separation, in subjecting the biomass to grinding and then to the action of acid hydrolysis at a sufficient temperature and pressure for the organic part of the waste to be substantially solubilised and converted to fermentescible sugars, and in fermenting the resulting product in order to cause the formation of alcohol and the production of residues for use as a nutrient, characterised in that:

a) the hydrolysis is carried out in the presence of sulphurous acid as the hydrolysis catalyst;

b) the resulting fermented product is filtered in order to separate off a filtrate and a residue; and

c) the filtrate is distilled, in a manner which is in itself known, in order to obtain a liquid fuel and a residue.

2. Process according to Claim 1, characterised in that the separation to which the solid waste is subjected comprises:

a) separation of the metallic materials;

b) separation of the friable and non-friable materials, the friable materials mainly consisting of brittle plastics, bones and glass and containing the biomass, and the non-friable materials mainly consisting of non-magnetic metals, leather, paper, fabric, cartilage and plastic and rubber films; and

c) separation of the abovementioned friable meterials and the biomass by flotation in water, this separation causing the hydrolysable material constituting the biomass to be immersed in water.

3. Process according to Claim 2, characterised in that the separation of the metallic materials mentioned in step a) of Claim 2 comprises a magnetic separation making it possible to recover the magnetic metals.

4. Process according to one of Claims 2 or 3, characterised in that step b) of Claim 2 is carried out by grinding and then screening the waste, the friable materials passing through the screen whereas the non-friable materials do not pass through.

5. Process according to one of Claims 1 to 4, characterised in that the residue from step b) of Claim 1 is subjected to sterilisation before being used to manufacture a nutrient.

6. Use of the residue from step b) of Claim 1 and/or the residue from step c) of Claim 1 in order to manufacture an animal feed or plant food.

7. Use of the liquid fuel from step c) of Claim 1 as a fuel for internal combustion engines.